# EUROPÄISCHE PATENTSCHRIFT

(11) **EP 0 939 107 B1**
(45) Veröffentlichungstag und Bekanntmachung des Hinweises auf die Patenterteilung: **02.11.2006**
(21) Anmeldenummer: 99103371.3
(22) Anmeldetag: 22.02.1999
(51) Int. Cl.: C08L 83/06, A61K 6/10

(54) **Bei Raumtemperatur aushärtende Silicon-Masse und ihre Verwendung**
Room temperature curable Silicone composition and its use
Composition de silicone durcissable à la température ambiante et son utilisation

(30) Priorität: 28.02.1998 DE 19808557
(43) Veröffentlichungstag der Anmeldung: 01.09.1999
(73) Patentinhaber: Heraeus Kulzer GmbH, 63450 Hanau (DE)
(72) Erfinder: Schwabe, Peter Dr., 51375 Leverkusen (DE); Freckmann, Michael, 50769 Köln (DE); Nehren, Klaus-Dieter, 41539 Dormagen (DE)
(74) Vertreter: Kühn, Hans-Christian

(56) Entgegenhaltungen:
- WO-A-99/62461
- DE-A- 3 636 974
- DE-A- 4 332 037
- US-A- 4 401 498
- US-A- 5 925 723

## Beschreibung

Die Erfindung betrifft eine bei Raumtemperatur durch Kondensation aushärtende Silicon-Masse aus Hydroxy-Gruppen aufweisendes Polyorganosiloxan und Füllstoff enthaltender Basis-Paste sowie Vernetzer und Katalysator aus Organometallverbindung enthaltender Aktivator-Komponente und die Verwendung der Silicon-Masse.

Die Erfindung betrifft besonders eine bei Raumtemperatur durch Kondensation aushärtende Silicon-Masse aus Hydroxy-Gruppen aufweisendes Polyorganosiloxan und Füllstoff enthaltender Basis-Paste sowie Vernetzer und Katalysator aus Organometallverbindung enthaltender Aktivator-Komponente zur Verwendung als dentale Silicon-Abformmasse.

Je nach Art der Vernetzung werden durch Kondensation vernetzende und durch Addition vernetzende Silicon-Abformmassen unterschieden (R. Marxkors /H. Meiners, Taschenbuch der zahnärztlichen Werkstoffkunde, München Wien: Carl Hanser Verlag, 1978; Ullmann's Encyclopedia of Industrial Chemistry, 5. Auflage, Weinheim; New York: VCH, Volume 8, 1987, 288).

Die in Form eines Zwei-Komponentensystems vorliegenden Silicon-Abformmassen bestehen - wenn sie zu den durch Kondensation vernetzenden gehören - aus Polydimethylsilanole oder andere Hydroxy-Polyorganosiloxane und Füllstoff enthaltender Basis-Paste sowie Vernetzer und Katalysator für die Polykondensation enthaltender Aktivator-Flüssigkeit oder -Paste. Nach dem kurz vor Gebrauch zu erfolgenden Vermischen der beiden Komponenten reagieren die Polydimethylsilanole mit dem üblicherweise aus Kieselsäureestern oder anderen Alkoxysilanen bestehenden Vernetzer durch Kondensation unter Kettenverlängerung, -verzweigung und -vernetzung, und es bilden sich für die Abformung sehr gut geeignete gummielastische Materialien.

Durch Kondensation vernetzende Silicon-Abdruckmassen sind zum Beispiel aus DE 1 153 169 B1, DE 26 44 193 A1, DE 34 06 233 A1, DE 36 36 974 A1 und DE 43 32 037 A1 bekannt.

DE 1 153 169 B1 bezieht sich auf ein Verfahren zur Herstellung von elastomeren Formteilen aus zwei getrennt vorliegenden, pastenartigen Massen, die vor dem Aushärten bei Raumtemperatur miteinander gemischt werden. Die eine der Massen enthält hydroxylendblockiertes Diorganopolysiloxan und Vernetzer, zum Beispiel Kieselsäureester oder Organowasserstoffpolysiloxane, die andere durch Triorganosiloxy-Gruppen endblockiertes Diorganopolysiloxan und den Kondensationskatalysator, zum Beispiel Dibutylzinndilacetat. Die Massen werden vor allem als Abdruck- oder Abdichtmassen für technische, künstlerische oder besonders für dentale Zwecke angewendet. Nachteilig ist, daß die das hydroxylendblockierte Diorganopolysiloxan und den Vernetzer enthaltende Masse in ihrer Wirkung bei Lagerung merklich nachläßt.

Aus DE 26 44 193 A1 sind pastöse Massen für bei Raumtemperatur vulkanisierbare Polyorganosiloxane bekannt, die neben vernetzenden Substanzen und Katalysatoren (Härtungskatalysatoren) als Verdickungsmittel 3 - 40 Gewichts-% aktive hydrophile Kieselsäure und gegebenenfalls bis zu 40 Gewichts-% inaktive Füllstoffe, zum Beispiel Quarzmehl oder Titandioxid, enthalten. Vernetzende Substanzen sind Ester von Kiesel- und Polykieselsäuren, Alkylalkoxy-, Arylalkoxy- oder Alkylalkanoyloxysilane. Ihre Menge beträgt 0,1 - 10 Gewichts-Teile, bezogen auf das Polyorganosiloxan. Katalysatoren sind carbonsaure Metallsalze, wie Dibutylzinndilaurat, Zinn(II)-octanoat, Bleilaurat, Kobaltnaphthenat und Tetraisopropyltitanat, oder Amine beziehungsweise Aminsalze, wie Hexylamin, Cyclohexylamin und Butylammoniumacetat. Sie werden in einer Menge zwischen 0,1 und 10 %, bezogen auf das Polyorganosiloxan, eingesetzt. Die pastösen Massen sind in feuchtigkeitsdichten Verpackungen lagerstabil und können ebenso wie die Polyorganosiloxan-Pasten in Tuben mit ausgewähltem Öffnungsdurchmesser gefüllt und über die Länge der aus den Tuben gepreßten Stränge dosiert werden. Die Durchmesser der Tubenöffnungen werden so gewählt, daß auf 100 Gewichts-Teile der Polyorganosiloxan-Pasten zwischen 3 und 40 Gewichts-Teile der pastösen Massen entfallen.

In DE 34 06 233 A1 werden für bei Raumtemperatur durch Kondensation oder Addition aushärtende pastenförmige Silicon-Massen bestimmte feinteilige anorganische Füllstoffe beschrieben, deren Teilchen mit Paraffinöl überzogen sind, eine mittlere Teilchengröße zwischen 1 und 25 Mikrometer aufweisen und aus Calciumcarbonat, Cristobalit oder Quarzmehl bestehen können. Die Silicon-Massen enthalten 30 - 90 Gewichts-% der Füllstoffe und werden bevorzugt in Abformmaterialien für dentale Zwecke verwendet, wobei kondensations- und additionsvernetzende Systeme unterschieden werden. Im ersten Fall enthalten die flüssigen oder pastenförmigen Aktivator-Komponenten ein carbonsaures Metallsalz und einen Kieselsäureester und die Silicon-Massen Polyorganosiloxane mit zwei oder mehr Hydroxy-Gruppen im Molekül.

In DE 43 32 037 A1 wird ein kondensationsvernetzendes Silicon zur Abformung in der Zahnmedizin vorgeschlagen, dessen Haupt- und Nebenkomponenten im Verhältnis 1 : 1 angemischt und im Volumenverhältnis 1 : 1 in Kammern von Doppelkartuschen abgefüllt werden können. Die Hauptkomponente besteht aus Hydroxypolysiloxan, pyrogener Kieselsäure, Calciumcarbonat, Wasser und Dibutylzinndilaurat, die Nebenkomponente aus Cristobalit, Siliconöl und Paraffinöl. Dieses Silicon enthält jedoch keinen Vernetzer und härtet daher nicht zu einem gummielastischen Material aus.

Während bei additionsvernetzenden Silicon-Abformmassen Zwei-Komponentensysteme in Form lagerfähiger Pasten, die über das Gewicht oder das Volumen vorzugsweise im Verhältnis 1 : 1 dosiert werden können, bekannt sind (siehe zum Beispiel EP 0 219 660 B1), fehlen in dieser Art zu dosierende und lagerfähige Vernetzer-haltige Systeme bei den kondensationsvernetzenden Silicon-Abformmassen.

Der Erfindung liegt daher die Aufgabe zugrunde, eine bei Raumtemperatur durch Kondensation aushärtende, aus den beiden Komponenten Basis-Paste und Aktivator-Komponente bestehende Silicon-Masse der eingangs charakterisierten Art zu finden, deren beide Komponenten lagerstabil sind, im gewünschten Mischungsverhältnis zueinander dosiert und in jedem Verhältnis homogen miteinander gemischt werden können. Die Aktivator-Komponente soll eine dünnflüssige bis pastenförmige Konsistenz besitzen und die Darbietung der Silicon-Masse in Tuben, in Schlauchbeuteln, die für die gemeinsame Verwendung mit Kartuschen bestimmt und beispielsweise aus DE 296 02 111 U1 bekannt sind, und vorzugsweise in Doppelkartuschen ermöglichen. Die Silicon-Masse soll sich besonders zur Verwendung als dentale Abformmasse eignen.

Doppelkartuschen sind Zweikammer-Vorrichtungen der zum Beispiel in EP 0 378 806 B1 beschriebenen Art zum Mischen miteinander reagierender Komponenten und Ausbringen der erhaltenen pastösen Mischungen. Vor Gebrauch wird der die Doppelkartuschen ursprünglich verschließende Stopfen entfernt und ein an seinem vorderen Ende mit einer Ausbringöffnung versehener statischer Mischer eingesetzt. Mit Doppelkartuschen lassen sich automatisch das richtige Mischungsverhältnis der zu mischenden Komponenten und die Homogenität der gemischten Pasten auf einfache Weise erreichen.

Die die Lösung der Aufgabe darstellende Silicon-Masse ist erfindungsgemäß dadurch gekennzeichnet, daß die Aktivator-Komponente zusätzlich ein Polyadditionsprodukt mit mindestens zwei Alkoxysilyl-Gruppen im Molekül enthält.

Unter Polyadditionsprodukten werden im Sinne der Erfindung die als Folge einer Polyaddition gewonnenen Produkte verstanden. Die Polyaddition ist eine Polyreaktion, bei der durch vielfach wiederholte Addition von bis- oder polyfunktionellen Edukten oder Monomeren Polymere aufgebaut werden (Römpp Chemie Lexikon, 9. Auflage, Stuttgart; New York: Georg Thieme Verlag, Band 5, 3508).

Die erfindungsgemäße Silicon-Masse hat sich besonders bewährt, wenn das Polyadditionsprodukt ein Ether-, Urethan-, Harnstoff- und Alkoxysilyl-Gruppen enthaltendes Polyadditionsprodukt mit einer überwiegend linearen Molekülstruktur, ausschließlich aliphatisch oder cycloaliphatisch gebundenen Ether-, Urethan- und Harnstoffsegmenten und einem mittleren Molekulargewicht von 800 - 20000,
a) einem Gehalt von Polyether-Gruppen von 25 - 90 Gewichts-%,
b) einem Gehalt von Urethan-Gruppen (-NH-CO-O-) von 0,5 - 10 Gewichts-%,
c) einem Gehalt von Harnstoff-Gruppen (-NH-CO-NH-) von 0,5 - 10 Gewichts-% und
d) Gruppen der Formel -NR-(CH₂)ₙ-SiR¹R²R³, worin n die Zahlen 1 - 6 darstellt, R Wasserstoff oder -(CH₂)ₙ-SiR¹R²R³ bedeutet, R¹, R², R³ unabhängig voneinander C₁-C₄-Alkoxy bedeuten, wobei der Gehalt der Alkoxysilyl-Gruppen -SiR¹R²R³ 1 - 25 Gewichts-% beträgt, ist.

Polyadditionsprodukte der bevorzugten Art und ihre Darstellung sind zum Beispiel aus DE 36 36 974 A1 bekannt. Nach DE 36 36 974 A1 werden die dort beschriebenen Ether-, Urethan- und Harnstoff-Gruppen enthaltenden Polyadditionsprodukte mit Alkoxysilyl-Gruppen einer überwiegend linearen Molekülstruktur mit ausschließlich (cyclo)aliphatisch gebundenen Ether-, Urethan- und Harnstoffsegmenten und einem mittleren Molekulargewicht von 800 - 20000 besonders in Form von Pasten zur Herstellung genauer Abformungen bezahnter, teilbezahnter und unbezahnter Kiefer und von Gipsmodellen eingesetzt. Für diesen Zweck werden Mischungen der Polyadditionsprodukte mit einem Vernetzungsmittel, insbesondere Tetraethoxysilan, zubereitet, die dann zur Herstellung von bei Raumtemperatur vernetzenden Abform- und Dubliermassen mit weiteren Komponenten versetzt werden.

Überraschenderweise wird durch die Mitverwendung der aus DE 36 36 974 A1 bekannten Polyadditionsprodukte in der üblicherweise aus Vernetzer und katalytisch wirksamer Organometallverbindung gebildeten Aktivator-Komponente eine aus Basis-Paste und Aktivator-Komponente bestehende Silicon-Masse geschaffen, die sich durch die sehr gute Hydrolysebeständigkeit und Lagerstabilität der Aktivator-Komponente auszeichnet. Die Konsistenz der Aktivator-Komponente läßt sich je nach Bedarf von dünnfließend bis pastenförmig einstellen. Basis-Paste und Aktivator-Komponente lassen sich sowohl manuell als auch automatisch genau dosieren und können in jedem Verhältnis homogen miteinander gemischt werden, so daß die erfindungsgemäße Silicon-Masse vorteilhaft auch in Verpackungen, die automatisches Abmessen, Dosieren und Mischen erlauben, dargeboten werden kann.

Für die erfindungsgemäße Silicon-Masse hat sich eine Aktivator-Komponente als besonders geeignet erwiesen, die 10 - 90 Gewichts-%, vorzugsweise 30 - 60 Gewichts-%, des Polyadditionsproduktes enthält. Die Menge an Polyadditionsprodukt richtet sich nach der gewünschten Konsistenz der Aktivator-Komponente, die je nach Bedarf dünnfließend bis pastenförmig sein kann. Die gewünschte Konsistenz ergibt sich aus der den praktischen Erfordernissen angepaßten Darbietungsform der Silicon-Masse.

Vernetzer und Katalysator bilden die weiteren Bestandteile der Aktivator-Komponente. Als Vernetzer können alle für diesen Zweck bekannten Kieselsäureester und anderen Alkoxysilane eingesetzt werden. Bevorzugt werden 1 - 16 C-Atome im Alkoxy-Rest aufweisende Alkoxysilane, die gegebenenfalls noch ethylenisch ungesättigte Gruppen im Molekül enthalten können, wie zum Beispiel Vinyltrimethoxysilan.

Geeignete Katalysatoren sind die für diesen Zweck an sich bekannten Organozinn-, -titan- und -zirkoniumverbindungen, besonders Dibutyl- und Dioctylzinnoxid und Dibutylzinndilaurat.

Die Mitverwendung anorganischer Füllstoffe, wie zum Beispiel von Kieselsäure, in der Aktivator-Komponente, ist zwar möglich, aber nicht unbedingt erforderlich.

Die Zusammensetzung der Basis-Paste weist keine Besonderheiten auf und entspricht der bekannter Silicon-Massen des kondensationsvernetzenden Typs. Neben dem Hydroxy-Gruppen aufweisenden Polyorganosiloxan enthält die Basis-Paste weiterhin an sich bekannte Füllstoffe, zum Beispiel Quarz, Cristobalit, Calciumcarbonat, Natriumsilicat, Calciumsilicat und/oder Glas in üblicher Menge und Teilchengröße, und gegebenenfalls Verarbeitungshilfsmittel, wie hydriertes Rizinusöl. Ein Füllstoff-Gehalt von 5 - 50 Gewichts-% hat sich besonders bewährt.

Die erfindungsgemäße Silicon-Masse eignet sich für den Formenbau, für Einbettungen und Beschichtungen und für ähnliche Anwendungen. Als besonders vorteilhaft erweist sich ihr Einsatz als dentale Abformmasse.

Zur näheren Erläuterung werden im folgenden für die erfindungsgemäße Silicon-Masse geeignete Beispiele einer Basis-Paste (Beispiele 1 und 2) und einer Aktivator-Komponente (Beispiel 3) beschrieben. Beispiel 4 bezieht sich auf die Beurteilung der Lagerstabilität der Aktivator-Komponente, Beispiel 5 auf die Prüfung der aus den Basis-Pasten A und B und der Aktivator-Komponente angemischten Silicon-Massen - Silicon-Massen I und II - nach der Norm ISO 4823:1992, in der die Anforderungen an zahnärztliche elastomere Abform- massen festgelegt werden.

### Beispiel 1

### Basis-Paste A

In einem Vakuum-Planetenmischer werden - in der angegebenen Reihenfolge - 20 Gewichts-% Hydroxy-Gruppen aufweisendes Polydimethylsiloxan mit einer Viskosität von 5000 mPa.s bei 23 °C, 51 Gewichts-% Hydroxy-Gruppen aufweisendes Polydimethylsiloxan mit einer Viskosität von 1000 mPa.s bei 23 °C, 24 Gewichts-% Calciumsilicat, 4 Gewichts-% pyrogene Kieselsäure und 1 Gewichts-% anorganischer Farbstoff bei Raumtemperatur und normalem Druck mit 50 U/min 30 Minuten lang miteinander zu einer Paste vermischt. Anschließend wird die Paste noch 5 Minuten lang im Vakuum entgast.

Die fertige Paste wird dann a) in Tuben, b) in Schlauchbeutel und c) in jeweils eine der beiden Kammern von Doppelkartuschen abgefüllt.

### Beispiel 2

### Basis-Paste B

In einem Vakuum-Planetenmischer werden - in der angegebenen Reihenfolge - 75 Gewichts-% Hydroxy-Gruppen aufweisendes Polydimethylsiloxan mit einer Viskosität von 2000 mPa.s bei 23 °C, 20 Gewichts-% eines Gemisches aus Natrium- und Calciumsilicat, 4 Gewichts-% hydriertes Rizinusöl und 1 Gewichts-% anorganischer Farbstoff bei Raumtemperatur und normalem Druck mit 50 U/min 30 Minuten lang miteinander zu einer Paste vermischt. Anschließend wird die Paste noch 5 Minuten lang im Vakuum entgast.

Die fertige Paste wird dann a) in Tuben, b) in Schlauchbeutel und c) in jeweils eine der beiden Kammern von Doppelkartuschen gefüllt.

### Beispiel 3

### Aktivator-Komponente

Wie in DE 36 36 974 A1, Beispiel 3, beschrieben, wird aus einem aus Propylenoxid, Ethylenoxid und Propylenglykol zubereiteten Polyether, Isophorondiisocyanat, Zinnoctanoat und 3-Aminotriethoxylsilan ein Polyadditionsprodukt mit einem Gehalt an Alkoxysilylgruppen von 5,96 Gewichts-% hergestellt. 72,5 Gewichts-% des Polyadditionsproduktes werden mit 27,5 Gewichts-% eines aus Vinyltrimethoxysilan und Dibutylzinnoxid durch einstündiges Erhitzen auf 120 °C unter Rückfluß und anschließendes Abkühlenlassen gewonnenen Präparats homogen miteinander vermischt. Die erhaltene Aktivator-Komponente wird a) in Tuben, b) in Schlauchbeutel und c) in die jeweils zweiten Kammern der Doppelkartuschen, deren erste Kammern bereits die Basis-Paste A beziehungsweise die Basis-Paste B enthalten, gefüllt.

### Beispiel 4

### Lagerstabilität

Zur Beurteilung der Lagerstabilität werden die Basis-Pasten A und B und die Aktivator-Komponente a) unmittelbar nach dem Abfüllen in die Verpackung und b) nach 60tägiger Lagerung der Verpackung bei Raumtemperatur und 80 % Luftfeuchtigkeit im Volumenverhältnis von 4 : 1 miteinander gemischt und die Gesamtverarbeitungszeiten der erhaltenen Silicon-Massen bestimmt. Die Gesamtverarbeitungszeiten betragen a) 2 Minuten und b) 2,15 Minuten, das heißt, die Wirksamkeit der Aktivator-Komponente wird durch die Lagerung praktisch nicht beeinflußt.

### Beispiel 5

### Prüfung nach der Norm ISO 4823:1992

Die Silicon-Massen I und II werden durch Vermischen von 4 Volumen-Teilen der Basis-Pasten A und B mit jeweils 1 Volumen-Teil der Aktivator-Komponente zubereitet und nach der Norm ISO 4823:1992 geprüft. Die Ergebnisse der Prüfung von Konsistenz, Gesamtverarbeitungszeit, Verformung unter Druck, Rückstellung nach Verformung und linearer Maßänderung werden in der Tabelle angegeben.

**Tabelle**

| Prüfung (ISO 4823:1992) | Silicon-Masse I | Silicon-Masse II |
|---|---|---|
| Konsistenz/Scheibendurchmesser [mm] | 37,5 | 37,0 |
| Gesamtverarbeitungszeit [s] | 120 | 120 |
| Verformung unter Druck [%] | 6,9 | 8,6 |
| Rückstellung nach Verformung [%] | 99,4 | 98,5 |
| Lineare Maßänderung [%] | 0,99 | 0,92 |

## Patentansprüche

1. Bei Raumtemperatur durch Kondensation aushärtende Silicon-Masse aus Hydroxy-Gruppen aufweisendes Polyorganosiloxan und Füllstoff enthaltender Basis-Paste sowie Vernetzer und Katalysator aus Organometallverbindung enthaltender Aktivator-Komponente, **dadurch gekennzeichnet, daß** die Aktivator-Komponente zusätzlich ein Polyadditionsprodukt mit mindestens zwei Alkoxysilyl-Gruppen im Molekül enthält.

2. Silicon-Masse nach Anspruch 1, **dadurch gekennzeichnet, daß** das Polyadditionsprodukt ein Ether-, Urethan-, Harnstoff- und Alkoxysilyl-Gruppen enthaltendes Polyadditionsprodukt mit einer überwiegend linearen Molekülstruktur, ausschließlich aliphatisch oder cycloaliphatisch gebundenen Ether-, Urethan- und Harnstoffsegmenten und einem mittleren Molekulargewicht Mn von 800 - 20000,
a) einem Gehalt von Polyether-Gruppen von 25 - 90 Gewichts-%,
b) einem Gehalt von Urethan-Gruppen (-NH-CO-O-) von 0,5 - 10 Gewichts-%,
c) einem Gehalt von Harnstoff-Gruppen (-NH-CO-NH-) von 0,5 - 10 Gewichts-% und
d) Gruppen der Formel -NR-(CH₂)ₙ-SiR¹R²R³, worin n die Zahlen 1 - 6 darstellt, R Wasserstoff oder -(CH₂)ₙ-SiR¹R²R³ bedeutet, R¹, R², R³ unabhängig voneinander C₁-C₄-Alkoxy bedeuten, wobei der Gehalt der Alkoxysilyl-Gruppen -SiR¹R²R³1 - 25 Gewichts-% beträgt, ist.

3. Silicon-Masse nach Anspruch 1 oder 2, **dadurch gekennzeichnet, daß** die Menge an Polyadditionsprodukt in der Aktivator-Komponente 10 - 90 Gewichts-% beträgt.

4. Silicon-Masse nach Anspruch 3, **dadurch gekennzeichnet, daß** die Menge an Polyadditionsprodukt in der Aktivator-Komponente 30 - 60 Gewichts-% beträgt.

5. Silicon-Masse nach einem der Ansprüche 1 bis 4, **dadurch gekennzeichnet, daß** die Aktivator-Komponente Vinyltrimethoxysilan als Vernetzer enthält.

6. Silicon-Masse nach einem der Ansprüche 1 bis 5, **dadurch gekennzeichnet, daß** die Aktivator-Komponente Dibutyl- oder Dioctylzinnoxid als Katalysator enthält.

7. Silicon-Masse nach einem der Ansprüche 1 bis 6, **dadurch gekennzeichnet, daß** die Basis-Paste 5 - 50 Gewichts-% des Füllstoffs enthält.

8. Silicon-Masse nach Anspruch 7, **dadurch gekennzeichnet, daß** die Basis-Paste Calciumsilicat als Füllstoff enthält.

9. Silicon-Masse nach einem der Ansprüche 1 bis 8, **gekennzeichnet durch** die Darbietung von Basis-Paste und Aktivator-Komponente in Tuben.

10. Silicon-Masse nach einem der Ansprüche 1 bis 8, **gekennzeichnet durch** die Darbietung von Basis-Paste und Aktivator-Komponente in Schlauchbeuteln.

11. Silicon-Masse nach einem der Ansprüche 1 - 8, **gekennzeichnet durch** die Darbietung von Basis-Paste und Aktivator-Komponente in Doppelkartuschen.

12. Verwendung der Silicon-Masse nach einem der Ansprüche 1 bis 11 zur Herstellung von dentalen Abformmassen.

## Claims

1. Silicone material curing at room temperature by condensation and comprising polyorganosiloxanes having hydroxyl groups and filler-containing base paste and crosslinking agent and catalyst comprising activator component containing organometallic compound, **characterized in that** the activator component additionally contains a polyadduct having at least two alkoxysilyl groups in the molecule.

2. Silicone material according to Claim 1, **characterized in that** the polyadduct is a polyadduct containing ether, urethane, urea and alkoxysilyl groups and having a predominantly linear molecular structure, excluding aliphatically or cycloaliphatically bonded ether, urethane and urea segments, and an average molecular weight Mn of 800-20 000,
a) a content of polyether groups of 25-90% by weight,
b) a content of urethane groups (-NH-CO-O-) of 0.5-10% by weight,
c) a content of urea groups (-NH-CO-NH-) of 0.5-10% by weight, and
d) groups of the formula -NR-(CH₂)ₙ-SiR¹R²R³, in which n represents the numbers 1-6, R denotes hydrogen or -(CH₂)ₙ-SiR¹R²R³, and R¹, R², R³, independently of one another, denote C₁-C₄-alkoxy, the content of alkoxysilyl groups - SiR¹R²R³ being 1-25% by weight.

3. Silicone material according to Claim 1 or 2, **characterized in that** the amount of polyadduct in the activator component is 10-90% by weight.

4. Silicone material according to Claim 3, **characterized in that** the amount of polyadduct in the activator component is 30-60% by weight.

5. Silicone material according to any of Claims 1 to 4, **characterized in that** the activator component contains vinyl trimethoxysilane as a crosslinking agent.

6. Silicone material according to any of Claims 1 to 5, **characterized in that** the activator component contains dibutyl- or dioctyltin oxide as a catalyst.

7. Silicone material according to any of Claims 1 to 6, **characterized in that** the base paste contains 5-50% by weight of filler.

8. Silicone material according to Claim 7, **characterized in that** the base paste contains calcium silicate as the filler.

9. Silicone material according to any of Claims 1 to 8, **characterized by** the provision of base paste and activator component in tubes.

10. Silicone material according to any of Claims 1 to 8, **characterized by** the provision of base paste and activator component in tubular bags.

11. Silicone material according to any of Claims 1-8, **characterized by** the provision of base paste and activator component in double cartridges.

12. Use of the silicone material according to any of Claims 1 to 11 for the preparation of dental impression compounds.

## Revendications

1. Composition de silicone durcissable par condensation à température ambiante, constituée d'une pâte de base contenant un polyorganosiloxane présentant des groupes hydroxy et une charge, ainsi que de composants activateurs contenant un agent de réticulation et un catalyseur d'un composé organo-métallique, **caractérisée en ce que** le composant activateur contient en outre, un produit de polyaddition avec au moins deux groupes alcoxysilyle dans la molécule.

2. Composition de silicone selon la revendication 1, **caractérisée en ce que** le produit de polyaddition est un produit de polyaddition contenant des groupes éther, uréthane, urée et alcoxysilyle avec une structure moléculaire essentiellement linéaire, des segments éther, uréthane et urée, exclusivement liés de manière aliphatique ou cycloaliphatique et un poids moléculaire moyen Mn de 800-20 000,
a) une teneur en groupes polyéther de 25-90% en poids,
b) une teneur en groupes uréthane (-NH-CO-O-) de 0,5-10% en poids,
c) une teneur en groupes urée (-NH-CO-NH-) de 0,5-10% en poids,
d) des groupes de la formule NR-(CH₂)ₙ-SiR¹R²R³, où n représente les nombres 1 à 6, R représente hydrogène ou -(CH₂)ₙ-SiR¹R²R³, R¹, R², R³ représentent indépendamment l'un de l'autre, alcoxy en C₁-C₄, où la teneur en groupes alcoxysilyle SiR¹R²R³ se situe dans la gamme allant de 1 à 25% en poids.

3. Composition de silicone selon la revendication 1 ou 2, **caractérisée en ce que** la quantité en produit de polyaddition dans le composant activateur se situe dans l'intervalle allant de 10 à 90% en poids.

4. Composition de silicone selon la revendication 3, **caractérisée en ce que** la quantité en produit de polyaddition dans le composant activateur se situe dans l'intervalle allant de 30 à 60% en poids.

5. Composition de silicone selon l'une des revendications 1 à 4, **caractérisée en ce que** le composant activateur contient le vinyltriméthoxysilane comme agent de réticulation.

6. Composition de silicone selon l'une des revendications 1 à 5, **caractérisée en ce que** le composant activateur contient l'oxyde de dibutyl- ou dioctylétain comme catalyseur.

7. Composition de silicone selon l'une des revendications 1 à 6, **caractérisée en ce que** la pâte de base contient 5 à 50% en poids de charge.

8. Composition de silicone selon la revendication 7, **caractérisée en ce que** la pâte de base contient le silicate de calcium comme charge.

9. Composition de silicone selon l'une des revendications 1 à 8, **caractérisée par** la présentation de la pâte de base et du composant activateur dans des tubes.

10. Composition de silicone selon l'une des revendications 1 à 8, **caractérisée par** la présentation de la pâte de base et du composant activateur dans des sachets-tubes.

11. Composition de silicone selon l'une des revendications 1 à 8, **caractérisée par** la présentation de la pâte de base et du composant activateur dans des cartouches doubles.

12. Utilisation de la composition de silicone selon l'une des revendications 1 à 11, pour la préparation de masses à mouler dentaires.
